# EUROPEAN PATENT APPLICATION

(11) **EP 4 417 697 A1**
(43) Date of publication of application: **21.08.2024**
(21) Application number: 23157204.1
(22) Date of filing: 17.02.2023
(51) Int. Cl.: C12N 15/90, A61K 31/713, C12N 9/22, C12N 15/11

(54) **MODULAR CONJUGATION SYSTEM**

(71) Applicant: Max-Planck-Gesellschaft zur Förderung der Wissenschaften e.V., 80539 München (DE)
(72) Inventor: Riesenberg, Stephan, 07743 Jena (DE); Kanis, Philipp, 04275 Leipzig (DE); Schaffer, Theresa, 04249 Leipzig (DE)
(74) Representative: Weickmann & Weickmann PartmbB

(57) **Abstract**

The present invention refers to a nucleic acid-peptide-complex comprising at least two subunits, wherein each subunit comprises a protein or peptide including a His-tag, and a nitrilotriacetic acid (NTA), iminodiacetic acid (IDA) or aminopolycarboxylic acid (APA)-modified nucleic acid, preferably a tris-NTA-modified nucleic acid, wherein the nucleic acids of the at least two subunits are configured to form a double-stranded oligonucleotide. Further, an oligonucleotide targeting complex comprising a protein or peptide including a His-tag, and a NTA, IDA or APA-modified nucleic acid, wherein the protein or peptide is selected from a CRISPR enzyme and variants thereof, a CRISPR base editor, a CRISPR activator, a CRISPR repressor, a CRISPR prime editor, or a combination thereof is disclosed.

## Description

The present invention refers to a modular conjugation system. Provided is a nucleic acid-peptide-complex comprising at least two subunits, wherein each subunit comprises a protein or peptide including a His-tag, and a nitrilotriacetic acid (NTA), iminodiacetic acid (IDA) or aminopolycarboxylic acid (APA)-modified nucleic acid, wherein the nucleic acids of the at least two subunits are configured to form a double-stranded oligonucleotide. This complex allows for non-covalent linkage of numerous proteins or peptides, such as enzymes, base editors, transcriptional activators, transcriptional repressors, and/or reverse transcriptases.

A further aspect of the invention is an oligonucleotide targeting complex comprising a protein or peptide including a His-tag, and a NTA, IDA or APA-modified nucleic acid, wherein the protein or peptide is selected from a CRISPR enzyme and variants thereof, a CRISPR base editor, a CRISPR activator, a CRISPR repressor, a CRISPR prime editor, or a combination thereof. Moreover, the present invention refers to the use of such oligonucleotide targeting complex for manufacturing a nucleic acid-peptide-complex according to the invention and a library of oligonucleotide targeting complexes according to the invention.

A further aspect of the invention is an *in vitro* or *in vivo* use of an oligonucleotide targeting complex, a nucleic acid-peptide-complex or a library according to the invention for CRISPR mediated oligonucleotide binding and/or editing. Further, a preparation comprising an oligonucleotide targeting complex, a nucleic acid-peptide-complex or a library according to the invention for use in therapy is disclosed.

### Background

CRISPR genome editing technology can generate a targeted DNA double-strand break in a desired gene sequence. This requires the introduction of a CRISPR enzyme such as Cas9 into target cells. Transfection of Cas9 plasmids is very inefficient, whereas transfection of recombinant Cas9 protein results in targeted DNA double-strand breaks in almost the entire cell population.

Cellular repair of the DNA break can lead to modification of the gene sequence. Often this repair is error-prone and can lead to knock-out of the gene. Thus, although the DNA break happens at a precise site, its repair is usually not precise. However, the cell can use an added DNA template corresponding to the cut gene sequence as a template for precise error-free repair. If additional desired mutations are inserted into the synthesized DNA template, they will be precisely integrated into the genome. However, this precise repair based on homology-directed repair (HDR) repair pathway is very inefficient because mostly the other defective repair pathways take place.

Low HDR efficiency and thus inefficient precise genome editing is a key problem of CRISPR genome editing technology. Consequently, research tools that dramatically increase HDR efficiency and other ways of precise editing or even exclusively enable those would be invaluable for users of CRISPR genome editing technology, and would save a lot of working time and money, as well as enabling new research approaches in the first place.

Still unsatisfactory is the low transfection efficiency of novel CRISPR enzymes and CRISPR fusion proteins, as they are only available to users as plasmids and not as much more efficient recombinant proteins. Recombinant CRISPR fusion proteins, such as CRISPR base editors, CRISPR activators, CRISPR repressors or CRISPR prime editors, are currently not commercially available.

Several studies have shown that covalent fusion of a DNA template to a Cas9 protein increases HDR efficiency by bringing the repair template into physical proximity to the DNA double-strand break. However, this requires transfecting Cas9 fusion proteins encoded on plasmids *in vivo* (which is highly inefficient) or performing complicated coupling steps *in vitro,* so this approach is not used in practice. Further, there are only commercial DNA matrices that contain chemical nucleotide modifications to reduce degradation by cellular nucleases.

Thus, it is an objective of the present invention to solve the problem of inefficient production and transfection of proteins and peptides e.g. suitable as genome editing systems. Further, the problem of unreliable and inefficient genome editing processes, particularly concerning the step of DNA break repair, is addressed.

### Summary of the Invention

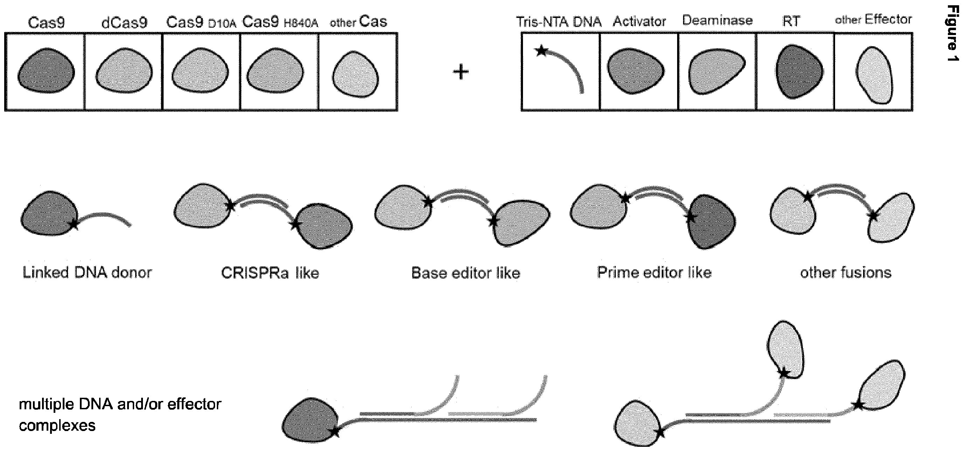

The present inventors have solved the problem of recombinant protein/peptide production and transfection as well the problem of error-prone genome editing by providing a nucleic acid-peptide-complex comprising at least two subunits including a protein or peptide with a His-tag and a carboxylic acid-modified nucleic acid. The His-tag can form a high affinity, non-covalent bond with the modified nucleic acid without the need for extensive coupling/purification steps. Further, the nucleic acid-peptide-complex may serve as a non-covalent conjugation system between at least two proteins and/or peptides based on an interaction of the nucleic acids of each subunit. Particularly, a subunit comprising a CRISPR enzyme such as Cas9 can form a complex with any desired peptide and/or protein by formation of a double-stranded oligonucleotide. This allows for the provision of a complex fusing at least two proteins and/or peptides in a non-covalent manner and yet in the required spatial orientation and proximity. The present conjugation system thus allows for a modular platform to connect different protein and/or peptide elements in a plug-and-play fashion (Figure 1).

Moreover, the present inventors have solved the problem of recombinant CRISPR/Cas protein/peptide production and transfection as well the problem of error-prone genome editing by providing an oligonucleotide targeting complex comprising a CRISPR protein or peptide with a His-tag, and a carboxylic acid-modified nucleic acid. The His-tag can form a high affinity, non-covalent bond with the modified nucleic acid without the need for extensive coupling/purification steps. By this means, the oligonucleotide targeting complex may serve as a non-covalent conjugation system between a CRISPR protein or peptide and a nucleic acid such as a DNA repair template. Particularly, an oligonucleotide targeting complex comprising a CRISPR enzyme such as Cas9 can form a complex with any desired CRISPR/Cas DNA repair template improving precise error-free repair in genome editing. The present complexes thus allows for the provision of a modular platform to connect different protein/peptides and nucleotides in a plug-and-play fashion (Figure 1).

A first aspect of the present invention relates to a nucleic acid-peptide-complex comprising at least two subunits, wherein each subunit comprises a protein or peptide including a His-tag, and a NTA, IDA or APA-modified nucleic acid, wherein the nucleic acids of the at least two subunits are configured to form a double-stranded oligonucleotide.

A further aspect of the present invention relates to a nucleic acid-peptide-complex comprising at least two subunits, wherein each subunit comprises a recombinant protein or peptide including a His-tag, and a NTA, IDA or APA-modified nucleic acid, wherein the nucleic acids of the at least two subunits are configured to form a (partially) double-stranded DNA or RNA.

A further aspect of the present invention relates to a nucleic acid-peptide-complex comprising at least two subunits, wherein each subunit comprises a protein or peptide including a His-tag, and a NTA, IDA or APA-modified nucleic acid, wherein the nucleic acids of the at least two subunits are configured to form a double-stranded oligonucleotide, and wherein at least one subunit comprises a CRISPR enzyme or a variant thereof including a CRISPR enzyme nickase, a catalytically inactive CRISPR enzyme variant and an optimized CRISPR enzyme variant.

A further aspect of the present invention relates to an oligonucleotide targeting complex comprising a protein or peptide including a His-tag, and NTA, IDA or APA-modified nucleic acid, wherein the protein or peptide is selected from a CRISPR enzyme and variants thereof, a CRISPR base editor, a CRISPR activator, a CRISPR repressor, a CRISPR prime editor, or a combination thereof.

A further aspect of the present invention relates to an oligonucleotide targeting complex comprising a recombinant protein or peptide including a His-tag, and NTA, IDA or APA-modified nucleic acid, wherein the protein or peptide is selected from a CRISPR enzyme and variants thereof, a CRISPR base editor, a CRISPR activator, a CRISPR repressor, a CRISPR prime editor, or a combination thereof, and wherein the nucleic acid comprises a DNA repair template, preferably a CRISPR/Cas DNA repair template.

A further aspect of the present invention relates to the use of an oligonucleotide targeting complex as described herein for manufacturing a nucleic acid-peptide-complex as described herein.

A further aspect of the present invention relates to a library comprising a plurality of oligonucleotide targeting complexes as described herein, comprising different protein or peptide sequences and a NTA, IDA or APA-modified nucleic acid, particularly a common DNA nucleic acid or complementary DNA nucleic acids.

A further aspect of the present invention relates to an *in vitro* or *in vivo* use of a nucleic acid-peptide-complex as described herein, an oligonucleotide targeting complex as described herein or a library of as described herein for CRISPR mediated oligonucleotide binding and/or editing, particularly genome editing.

A further aspect of the present invention relates to a preparation comprising a nucleic acid-peptide-complex as described herein, an oligonucleotide targeting complex as described herein, or a library as described herein for use in therapy including human medicine and veterinary medicine.

### Detailed description

The present inventors have provided means for increasing the efficacy of genome editing, particularly CRISPR-mediated genome editing, by designing a nucleic acid-peptide-complex capable of arranging at least two proteins and/or peptides in a desired spatial orientation and proximity. Further, production and use of the nucleic acid-peptide-complex does not require complicated and time-consuming covalent coupling steps or ineffective plasmid transfection. In particular, the present inventors have engineered a nucleic acid-peptide-complex comprising at least two subunits, wherein each subunit comprises a protein or peptide including a His-tag, and a nitrilotriacetic acid (NTA), iminodiacetic acid (IDA) or aminopolycarboxylic acid (APA)-modified nucleic acid, wherein the nucleic acids of the at least two subunits are configured to form a double-stranded oligonucleotide.

The term "nucleic acid-peptide-complex" includes complexes including only peptides, complexes including only proteins, and complexes including both proteins and peptides. The nucleic acid-peptide-complex of the invention is formed of at least two subunits. In one embodiment, the nucleic acid-peptide-complex comprises 2, 3, 4 or more subunits. In a preferred embodiment, the nucleic acid-peptide-complex comprises 2, 3, or 4 subunits.

The at least two subunits interact by the nucleic acids comprised in each subunit. Preferably, the nucleic acids of the at least two subunits form a double-stranded oligonucleotide. The term "double-stranded oligonucleotide" refers to at least two oligonucleotide molecules, such as DNA or RNA molecules, arranged such that they form a double strand by non-covalent linkage (such as hydrogen bonds) over at least a part of each oligonucleotide molecule. In one embodiment, a double strand is formed over the entire length of each oligonucleotide molecule (entirely double-stranded molecule). In another embodiment, a double strand is formed over the entire length of at least one of the oligonucleotide molecules and over a part of the other oligonucleotide molecules, or over a part of each oligonucleotide molecule (partially double-stranded molecule).

The nucleic acids may form a partially double-stranded oligonucleotide or an entirely double-stranded oligonucleotide. In one embodiment, the complex consists of two subunits and the double-stranded oligonucleotide is formed by the first nucleic acid and the second nucleic acid, particularly in the form of a partially double-stranded oligonucleotide.

In one embodiment, the nucleic acids are DNA molecules, preferably single stranded DNA (ssDNA) molecules, and particularly complementary ssDNA. The complementary ssDNA molecules may be partially complementary or complementary over the entire length of at least one ssDNA molecule. The DNA molecules of the at least two subunits may thus form a partially double-stranded DNA or an entirely double-stranded DNA. In a particularly preferred embodiment, the complex comprises two subunits with complementary ssDNA forming a partially double-stranded DNA.

In another embodiment, the nucleic acids are RNA molecules, preferably single stranded RNA (ssRNA) molecules, and particularly complementary ssRNA molecules. The complementary ssRNA molecules may be partially complementary or complementary over the entire length of at least one ssRNA molecule. The RNA molecules of the at least two subunits may thus form a partially double-stranded RNA or an entirely double-stranded RNA. In a particularly preferred embodiment, the complex comprises two subunits with complementary ssRNA forming a partially double-stranded RNA.

The nucleic acid may be any DNA or RNA molecule including modified DNA/RNA, such as chemically modified DNA/RNA including phosphorothioate bonds, for example, or base modified DNA/RNA including locked nucleic acids, 2'-O-methoxy-ethly bases, 2'-O-methyl RNA bases, and/or fluoro bases, for example, or any other modification.

In certain embodiments, at least one nucleic acid comprises a DNA repair template, preferably a CRISPR/Cas DNA repair template. A DNA repair template particularly serves to support HDR repair. DNA repair templates are well known in the art and particularly comprise a linear or circular DNA template sequence, which is optionally modified. Suitable modifications comprise chemical modification such as phosphorothioate bonds for improved stability of the DNA repair template, or terminal protection such as by PEG.

In some embodiments, the nucleic acid-peptide-complex may be immobilized on a substrate. The term "substrate" refers to a structure to which the complex can be coupled reversibly or irreversibly and includes, for example, planar matrices or beads. Suitable substrates are made of glass, silica, or polymeric resins. In a preferred embodiment, the nucleic acid-peptide-complex is not immobilized on a substrate.

The modified nucleic acids are independently selected from NTA, IDA and APA-modified nucleic acids. In a preferred embodiment, the nucleic acid-peptide-complex comprises only one type of nucleic acid modification, i.e. NTA, IDA or APA-modified nucleic acids.

Preferably, each modified nucleic acid is a tris-NTA-modified nucleic acid, a tris-IDA-modified nucleic acid or a tris-APA-modified nucleic acid, preferably a tris-NTA-modified nucleic acid.

Nitrilotriacetic acid (NTA) modification of nucleic acids is well known to a person skilled in the art. Particularly, NTA modification includes linkage of a maleimide linker bound to nitriloacetic acid (maleimide-NTA) to a nucleic acid having a thiol group (Goodman et al., ChemBioChem 2009, 10, 1551-1557, incorporated herein by reference). The structure of a NTA modified nucleic acid may be:

NTA-(C₃-C₁₀ alkyl)-[NHCO-(C₁-C₁₀ alkyl)]ₙ-maleimid nucleic acid

with n selected from an interger of 0-5.

Optionally, an additional linker L might be position between the maleimid moiety and the nucleic acid:

NTA-(C₃-C₁₀ alkyl)-[NHCO-(C₁-C₁₀ alkyl)]ₙ-maleimid-L-nucleic acid

with n selected from an interger of 0-5. Suitable linkers are C₂-C₁₀ carboxylic acid amides, preferably linear C₄-C₈ carboxylic acid amides, and C₂-C₁₀ alkyl, preferably linear C₂-C₆ alkyl, for example.

The structure of a NTA modified nucleic acid is preferably as shown in formula 1 and formula 2.

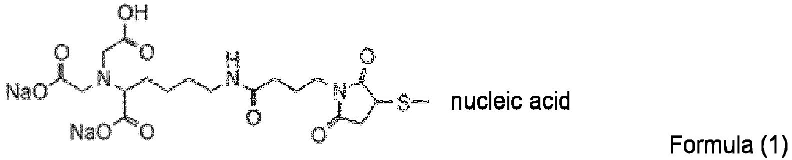

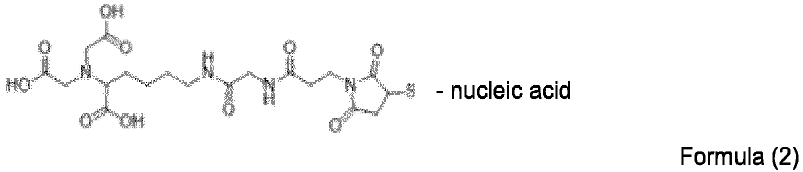

NTA might be present as protonated acid, partially protonated acid or any pharmaceutically acceptable salt thereof, such as a sodium or potassium salt.

Iminodiacetic acid (IDA) modification of nucleic acids is well known in the art. Preferably, IDA modification includes direct linkage of iminodiacetic acid to the nucleic acid. Alternatively, a linker is positioned between the IDA ligand and the nucleic acid, such as C₂-C₁₀ alkyl, preferably linear C₂-C₆ alkyl.

Aminopolycarboxylic acid (APA) modification of nucleic acids may include modification by one or more aminopolycarboxylic acid molecules, for example 2-52 aminopolycarboxylic acid molecules. Each aminopolycarboxylic acid molecule comprise at least two carboxylic acid moieties. For example, a suitable aminopolycarboxylic acid molecule comprises 2-10, preferably 3-6 carboxylic acid moieties. Preferably, the APA comprises at least one tetradentate aminopolycarboxylic acid (having 4 carboxylic acid moieties) and/or at least one pentadentate aminopolycarboxylic acid (having 5 carboxylic acid moieties).

Preferred aminopolycarboxylic acid molecules are selected from ethylenediaminetetraacetic acid (EDTA), diethylenetriaminepentaacetic acid (DTPA), triethylenetetraminehexaacetic acid (TTHA), ethylenediamine-N,N'-disuccinic acid (EDDS), ethylene glycol-bis(β-aminoethyl ether)-N,N,N',N'-tetraacetic acid (EGTA), 2,2',2",2‴-(1,4,7,10-tetraazacyclododecane-1,4,7,10-tetrayl)tetraacetic acid (DOTA), 1,4,7-triazacyclononane-1,4,7-triacetic acid (NOTA) or a combination thereof. Suitable APA compounds are disclosed in WO 2017/046625 and WO 2020/109162, for example, which are herein incorporated by reference. Moreover, a suitable APA modification is the INDIGO ligand available by Cube Biotech (Monheim, Germany).

The nucleic acid-peptide-complex can comprise any type of protein and peptide. In a preferred embodiment, the complex comprises at least one recombinant protein or peptide, preferably each protein/peptide is a recombinant protein or recombinant peptide, respectively.

Each protein and each peptide may independently be selected from an enzyme such as a CRISPR enzyme and variants thereof, a base editor such as a CRISPR base editor, a transcriptional activator such as a CRISPR activator, a transcriptional repressor such as a CRISPR repressor, a reverse transcriptase such as a CRISPR prime editor, an epigenic modulator, a DNA repair modulator, a splicing modulator, a fluorescent protein or a combination thereof.

In certain embodiments, the nucleic acid-peptide-complex comprises an enzyme. Suitable enzymes are CRISPR enzymes, transcription activator-like effector-based nucleases (TALENs), zinc finger nuclease proteins, Argonaute of the bacterium Thermus thermophiles (TtAgo), recombinases, or meganucleases, for example.

In a preferred embodiment, at least one subunit comprises a targetable oligonucleotide binding protein, wherein the targetable oligonucleotide protein is preferably selected from a CRISPR enzyme and variants thereof including a CRISPR enzyme nickase, a catalytically inactive CRISPR enzyme variant and an optimized CRISPR enzyme variant. The term "targetable oligonucleotide binding protein" refers to a protein that can be targeted to an oligonucleotide sequence of interest and is capable of binding to it.

In certain embodiments, the CRISPR enzyme and variants thereof are selected from a Cas enzyme, a Cas nickase, a catalytically inactive Cas variant and an optimized Cas variant, preferably Cas9, dCas9, Cas9-D10A, Cas9-H840A, Cas12a, Cas12b, Cas12c, Cas12d, Cas12e, Cas12f, Cas12g, Cas12h, Cas12i, Cas12j, Cas12k, Cas13a, Cas13b, Cas13c, Cas13d, Cas14, or other oligonucleotide guided nucleases, e.g. engineered non-naturally occurring nucleases such as those disclosed in US9,982,279, the content of which is incorporated herein by reference, including any recombinant variants thereof.

Preferably, the at least one subunit comprises a Cas9 or any recombinant variant thereof. The Cas9 enzyme may be a *Streptococcus,* e.g. a S. *pyogenes* or *Lactobacillus* Cas9 enzyme as described by Briner et al. (Mol Cell 2014, 56, 333-339), the content of which is herein incorporated by reference, including any recombinant variant thereof. In particular, the term "Cas enzyme" includes optimized Cas enzymes, e.g. Cas enzymes resulting in less off-target events such as R691A Cas9-Hifi and/or having a higher catalytic efficacy such as K918N SpyCECas9. Further, the term "Cas enzyme" as used herein includes a catalytically fully active Cas nuclease capable of creating a double-strand break in a DNA template, a catalytically partially active Cas nickase capable of creating a single-strand break in a double stranded DNA template, e.g. Cas9 D10A or Cas9 H840A, and a catalytically inactive Cas enzyme, e.g. dCas9. Further, the term "Cas enzyme" includes split-fusion versions of the above enzymes, e.g. split-fusion versions of Cas9 or Cas9 D10A.

In certain embodiments, the nucleic acid-peptide-complex comprises a base editor. A base editor is capable of providing a point mutation in a target nucleic acid by exchanging a single base. For example, the base editor may exchange C by U (cytosine deaminase base editor). U is subsequently converted to T through base excision repair, creating an overall C to T exchange (or a G to A exchange on the opposite strand). In another example, the base editor may exchange A by I (adenine deaminase base editor). I is subsequently converted to G through base excision repair, creating an overall A to G exchange (or a T to C exchange on the opposite strand). Preferably, the nucleic acid-peptide-complex comprises a CRISPR base editor, preferably a cytosine deaminase base editor, such as APOBEC, PmCDA1 or AID, an adenine deaminase base editor, such as TadA, or a combination thereof.

In certain embodiments, the nucleic acid-peptide-complex comprises a transcriptional activator. A transcriptional activator is a protein that increases transcription of a gene or set of genes. Transcriptional activators are considered to have positive control over gene expression, as they function to promote gene transcription and, in some cases, are required for the transcription of genes to occur. Transcriptional activator proteins comprise two main domains: a DNA-binding domain that binds to a nucleic acid sequence, such as DNA, specific to the activator, and an activation domain that functions to increase gene transcription by interacting with other molecules. Preferably, the nucleic acid-peptide-complex comprises a CRISPR activator, such as VP64, p65, HSF1, RTA, Tet1, p300, PRDM9, or a combination thereof.

In certain embodiments, the nucleic acid-peptide-complex comprises a transcriptional repressor. Transcriptional repressors are proteins that bind to specific sites on the target DNA and prevent transcription of nearby genes, e.g. by inhibiting the initiation of transcription. There are numerous ways of action known for transcriptional repressors. For example, the repressor binding site on the DNA overlaps the promotor, thereby preventing RNA polymerase (RNAP) binding required for transcription; the repressor binding site on the DNA overlaps the activator binding site, thereby preventing RNAP binding or action; the repressor binds to multiple sites and interacts to cause DNA looping, thereby preventing RNAP binding or action, for example. Preferably, the nucleic acid-peptide-complex comprises a CRISPR repressor, preferably a KRAB domain, SALL1, SDS3, ZIM3, MeCP2, DNMT3A, LSD1, or a combination thereof.

In certain embodiments, the nucleic acid-peptide-complex comprises a reverse transcriptase (RT). A reverse transcriptase is a multifunctional enzyme that may provide RNA- and/or DNA-dependent DNA polymerase activity, and is capable for reverse transcription of single-stranded RNA into double-stranded DNA. Preferably, the nucleic acid-peptide-complex comprises a CRISPR prime editor, preferably PE5max, M-MLV reverse transcriptase, or a combination thereof. PE5max may be used in combination with dominant negative MLH1 to further enhance prime genome editing (Chen et al., Cell 2021, 184, 5635-5652, which is incorporated herein by reference).

In certain embodiments, the nucleic acid-peptide-complex comprises an epigenic modulator. Epigenic modulators can modulate epigenic mechanisms, such as chromatin remodeling, DNA methylation status, and histone acetylation. Epigenic modulators according to the invention include epigenic repressors and epigenic activators. Suitable epigenic repressors are DNMT3A, LSD1, protein domains that are sufficient to act as a repressor or a combination thereof. Suitable epigenic activators are Tet1, p300, PRDM9 or a combination thereof.

In certain embodiments, the nucleic acid-peptide-complex comprises a DNA repair modulator. DNA repair modulators can modulate DNA repair and preferably facilitate precise DNA repair. Suitable DNA repair modulators are CtiP and its orthologs, TREX2, POLD3, other DNA repair pathway proteins, or combinations thereof.

In certain embodiments, the nucleic acid-peptide-complex comprises a splicing modulator. Splicing modulators can modulate RNA splicing. Suitable splicing modulators are RNA splicing modulators such as RBFOX1.

In certain embodiments, the nucleic acid-peptide-complex comprises a fluorescent protein. Suitable fluorescent proteins are the blue fluorescent protein (BFP; UniProt P80893), the green fluorescent protein (GFP; UniProt P42212), the red fluorescent protein (RFP; UniProt Q9U6Y8) or any other fluorescent protein suitable for oligonucleotide or chromatin labelling.

In certain embodiments, one subunit of the nucleic acid-peptide-complex comprises a CRISPR enzyme protein and a second subunit comprises a protein or peptide selected from a CRISPR base editor, a CRISPR activator, a CRISPR repressor, a CRISPR prime editor, an epigenic modulator, a DNA repair modulator, a splicing modulator, or a fluorescent protein.

In a preferred embodiment, the nucleic acid-peptide-complex comprises Cas9-H840A and a reverse transcriptase, preferably CRISPR prime editor M-MLV RT.

In another preferred embodiment, the nucleic acid-peptide-complex comprises dCas9 or Cas9-D10A and a CRISPR cytosine deaminase base editor, preferably APOBEC or PmCDA1.

In still another preferred embodiment, the nucleic acid-peptide-complex comprises dCas9 or Cas9-D10A and a CRISPR adenine deaminase base editor, preferably TadA.

In still another preferred embodiment, the nucleic acid-peptide-complex comprises dCas9 or Cas9-D10A and base editor AID (random base editor).

In still another preferred embodiment, the nucleic acid-peptide-complex comprises dCas9 or Cas9D10A and a CRISPR repressor, preferably KRAB and/or ZIM3 and/or MeCP2 and/or SALL1 and/or SDS3.

In still another preferred embodiment, the nucleic acid-peptide-complex comprises dCas9 and a CRISPR activator, preferably VP64 and/or p65 and/or HSF1 and/or RTA.

In still another preferred embodiment, the nucleic acid-peptide-complex comprises dCas9 or Cas9D10A and an epigenetic repressor, preferably DNMT3A and/or LSD1.

In still another preferred embodiment, the nucleic acid-peptide-complex comprises dCas9 and an epigenetic activator, preferably Tet1 and/or p300 and/or PRDM9.

In still another preferred embodiment, the nucleic acid-peptide-complex comprises dCas9 and a fluorescent protein, preferably BFP or GFP or RFP.

In still another preferred embodiment, the nucleic acid-peptide-complex comprises Cas9 or Cas9D10A and a DNA repair modulator, preferably CtiP or TREX2 or POLD3.

In still another preferred embodiment, the nucleic acid-peptide-complex comprises dCas13 and a RNA base editor, preferably ADAR.

In still another preferred embodiment, the nucleic acid-peptide-complex comprises dCas9 and a RNA splicing modulator, preferably RBFOX1.

In still another preferred embodiment, the nucleic acid-peptide-complex comprises dCas9 and a restriction endonuclease, preferably Fok1 allowing for high specificity cleavage with dual targeting.

In still another preferred embodiment, the nucleic acid-peptide-complex comprises Cas9-H840A and a reverse transcriptase, preferably M-MLV RT, and an integrase, preferably Bxb (prime editor integrase system). Particularly, the nucleic acid-peptide-complex comprises Cas9-H840A, reverse transcriptase M-MLV and integrase Bxb.

In still another preferred embodiment, the nucleic acid-peptide-complex comprises dCas9 and dimerizable protein domains such as ABI1 and PYL1 for chromatin topology manipulation.

In still another preferred embodiment, the nucleic acid-peptide-complex comprises dCas9 and APEX2 for proteome profiling.

The proteins and peptides of the present invention may be prepared by known methods including chemical synthesis, e.g. solid phase synthesis, or recombinant expression.

The nucleic acids of the present invention may be prepared by known methods including chemical synthesis, e.g. solid phase synthesis, or transcription *in vitro* or *in vivo. In vitro* transcription may be performed with a suitable polymerase, e.g. a bacteriophage RNA polymerase such as T7 or SP6 RNA polymerase. *In vivo* transcription may be performed by introducing a nucleic acid template into a cell and expressing it therein. The nucleic acid template may be introduced as an expression vector such as a plasmid in operative linkage with appropriate expression control elements for transient or stable expression in a cell.

Suitable transfection techniques for introducing proteins, peptides and/or nucleic acids into cells, e.g. eukaryotic cells, are well known in the art and include lipofection, electroporation, e.g. nucleofection, Ca-phosphate or virus-based methods.

In certain preferred embodiments, at least one subunit is a nucleic acid-peptide-complex and preferably each of the at least two subunits is a nucleic acid-peptide-complex. A complex may be formed between the His-tag carried by the protein or peptide and at least one carboxylic acid (such as acetic acid) carried by the nucleic acid. A complex may be formed by interaction with a polyvalent metal ion, preferably a divalent metal ion. A suitable metal ion is a Ni(II)-ion, a Co(II)-ion, a Zn(II)-ion, a Cu(II)-ion, or a Fe(II)-ion, preferably Ni²⁺. For example, each subunit may be a His-tag/Ni²⁺/NTA complex, each subunit may be a His-tag/Ni²⁺/IDA complex or each subunit may be a His-tag/Ni²⁺/APA complex.

Synthesis of a nucleic acid-peptide-complex according to the invention may include modifying nucleic acid molecules with NTA, IDA or APA, hybridizing the modified nucleic acids, and mixing the hybridized and modified nucleic acids with polyvalent metal ions, such as Ni²⁺, and proteins and/or peptides. Alternatively, synthesis of a nucleic acid-peptide-complex according to the invention may include modifying nucleic acid molecules with NTA, IDA or APA, mixing the modified nucleic acids with the respective proteins or peptides and polyvalent metal ions, such as Ni²⁺, to obtain complex subunits, and mixing the complex subunits to obtain a nucleic acid-peptide-complex.

Each subunit of the nucleic acid-peptide-complex comprises a protein or peptide inculding a His-tag. Each His-tag may be independently selected from a His-tag attached to the N-terminal of the respective protein or peptide, the C-terminal of the respective protein or peptide or an internal His-tag. An internal His-tag is arranged in the protein/peptide sequence and is spaced part from the N-terminal and the C-terminal of the protein /peptide by a predetermined number of one amino acids.

In one embodiment, each His-tag of the nucleic acid-peptide-complex is an N-terminal His-tag. In another embodiment, each His-tag of the nucleic acid-peptide-complex is a C-terminal His-tag.

The His-tag may be any His-tag and preferably comprises 2-14 His residues, more preferably 4-10 His residues. The His-residues of the His-tag may be arranged in a consecutive manner or in an alternating manner with at least one further amino acid. Suitable alternating amino acids are any known amino acids other than His and are preferably selected from aspartic acid, glutamic acid, proline, alanine, glycine, valine, serine, leucine, isoleucine, threonine, lysine, glutamine, and/or asparagine, more preferably lysine, glutamine, asparagine and/or glycine.

In certain embodiments, the His-tag comprises a spacer positioned between the protein or peptide and the His residues. The space can have a length of 1-50 amino acids, preferably 1-20 amino acids. The amino acids of the linker may be any amino acids other than His and are preferably selected form glycine, asparagine, alanine, aspartic acid, tyrosine, isoleucine, proline, threonine, serine, glutamic acid, lysine or combinations thereof. Examples of a linker sequence are Asp-Tyr-Asp-Ile-Pro-Thr-Thr (SEQ ID NO: 1), Gly-Gly-Gly-Gly-Gly-Gly-Gly (SEQ ID NO: 2), Gly-Gly-Gly-Gly-Ser (SEQ ID NO: 3), and Glu-Ala-Ala-Ala-Lys (SEQ ID NO: 4).

The His-tag may be independently selected from Hise (SEQ ID NO: 5), His₈ (SEQ ID NO: 6), His₁₀ (SEQ ID NO: 7), His₁₄ (SEQ ID NO: 8), (HisGln)₆ (SEQ ID NO: 9), (HisAsn)₆ (SEQ ID NO: 10), (HisGly)₅HisGln (SEQ ID NO: 11), a His-tag disclosed in US 5,594,115 (enclosed herein by reference), a His-tag disclosed in US 7,176,298 (enclosed herein by reference), or a His-tag disclosed in US 2004/029781 (enclosed herein by reference), for example. Preferably, the His-tag is independently selected from His₆, His₆ and His₁₀. The His-tag of the subunits of the nucleic acid-peptide-complex may be identical or different.

The nucleic acid-peptide-complex of the present invention may be a gene editing complex. Gene editing may comprises introducing a staggered cut into the doubled-stranded genome of a target cell or target organism, introducing a blunt-ended cut into the double-stranded genome of a target organism, insertion of a predetermined nucleotide sequence in the genome of a target cell or target organism, deletion of a predetermined nucleotide sequence in the genome of a target cell or target organism, modification of a predetermined nucleotide sequence in the genome of a target cell or target organism and combinations thereof. The nucleic acid-peptide-complex is intended for use in any type of genome editing including multiplexed genome editing on both chromosomes both in non-medical applications and in medical applications.

In some embodiments, the nucleic acid-peptide-complex of the invention comprises an oligonucleotide targeting complex as described herein as a subunit. In some embodiments, each of the subunits is an oligonucleotide targeting complex as described herein.

In a further aspect, the present inventors have provided means for increasing the efficacy of genome editing, particularly CRISPR-mediated genome editing, by designing an oligonucleotide targeting complex capable of arranging a protein or peptide in a desired spatial orientation and proximity to a nucleic acid. Further, production and use of the oligonucleotide targeting complex does not require complicated and time-consuming covalent coupling steps or ineffective plasmid transfection. In particular, the present inventors have engineered an oligonucleotide targeting complex comprising a protein or peptide including a His-tag and a nitrilotriacetic acid (NTA), iminodiacetic acid (IDA) or aminopolycarboxylic acid (APA)-modified nucleic acid, wherein the protein or peptide is selected from a CRISPR enzyme and variants thereof, a CRISPR base editor, a CRISPR activator, a CRISPR repressor, a CRISPR prime editor, or a combination thereof.

The term "oligonucleotide targeting complex" refers to a complex, which interacts with any DNA or RNA template, particularly a double-stranded DNA template or a single-stranded DNA template or a single-stranded RNA template, e.g. an oligonucleotide, a plasmid, a chromosome *in vitro* and *in vivo.* Interaction may include binding, cleavage, activation, repression, base editing, genome editing, epigenome editing and/or prime editing. The structural elements of the oligonucleotide targeting complex may be as defined for the nucleic acid-peptide-complex above.

The oligonucleotide targeting complex of the invention comprises a protein or peptide including a His-tag. The His-tag is as defined herein. Specifically, the His-tag may be selected from a His-tag attached to the N-terminal of the protein or peptide, a His-tag attached to the C-terminal of the protein or peptide, or an internal His-tag.

The His-tag may be any His-tag and preferably comprises 2-14 His residues, more preferably 4-10 His residues. The His residues of the His-tag may be arranged in a consecutive manner or in an alternating manner with at least one further amino acid. Suitable alternating amino acids are any known amino acids other than His and are preferably selected from aspartic acid, glutamic acid, proline, alanine, glycine, valine, serine, leucine, isoleucine, threonine, lysine, glutamine, and/or asparagine, more preferably lysine, glutamine, asparagine and/or glycine.

In certain embodiments, the His-tag comprises a spacer positioned between the protein or peptide and the His residues. The space can have a length of 1-50 amino acids, preferably 1-20 amino acids. The amino acids of the linker may be any amino acids other than His and are preferably selected form glycine, asparagine, alanine, aspartic acid, tyrosine, isoleucine, proline, threonine, serine, glutamic acid, lysine or a combination thereof. Examples of a linker sequence are Asp-Tyr-Asp-Ile-Pro-Thr-Thr (SEQ ID NO: 1), Gly-Gly-Gly-Gly-Gly-Gly-Gly (SEQ ID NO: 2), Gly-Gly-Gly-Gly-Ser (SEQ ID NO: 3), and Glu-Ala-Ala-Ala-Lys (SEQ ID NO: 4).

The His-tag may be selected from Hise(SEQ ID NO: 5), His₈ (SEQ ID NO: 6), His₁₀ (SEQ ID NO: 7), His₁₄ (SEQ ID NO: 8), (HisGln)s (SEQ ID NO: 9), (HisAsn)₆ (SEQ ID NO: 10), (HisGly)₅HisGln (SEQ ID NO: 11), a His-tag disclosed in US 5,594,115 (enclosed herein by reference), a His-tag disclosed in US 7,176,298 (enclosed herein by reference), or a His-tag disclosed in US 2004/029781 (enclosed herein by reference), for example. Preferably, the His-tag is selected from Hiss, Hise and His₁₀.

The protein or peptide is selected from a CRISPR enzyme and variants thereof, a CRISPR base editor, a CRISPR activator, a CRISPR repressor, a CRISPR prime editor, or a combination thereof. In a preferred embodiment, the protein or peptide is a recombinant protein or recombinant peptide.

In certain embodiments, the oligonucleotide targeting complex comprises a CRISPR enzyme and variants thereof including a CRISPR enzyme nickase, a catalytically inactive CRISPR enzyme variant and an optimized CRISPR enzyme variant.

In certain embodiments, the CRISPR enzyme and variants thereof are selected from a Cas enzyme, a Cas nickase, a catalytically inactive Cas variant and an optimized Cas variant, preferably Cas9, dCas9, Cas9-D10A, Cas9-H840A, Cas12a, Cas12b, Cas12c, Cas12d, Cas12e, Cas12f, Cas12g, Cas12h, Cas12i, Cas12j, Cas12k, Cas13a, Cas13b, Cas13c, Cas13d, Cas14, or other oligonucleotide guided nucleases, e.g. engineered non-naturally occurring nucleases such as those disclosed in US9,982,279, the content of which is incorporated herein by reference, including any recombinant variants thereof.

In a preferred embodiment, the CRISPR enzyme is Cas9 or any recombinant variant thereof. The Cas9 enzyme may be a *Streptococcus,* e.g. a S. *pyogenes* or *Lactobacillus* Cas9 enzyme as described by Briner et al. (Mol Cell 2014, 56, 333-339), the content of which is herein incorporated by reference, including any recombinant variant thereof. In particular, the term "Cas enzyme" includes optimized Cas enzymes, e.g. Cas enzymes resulting in less off-target events such as R691A Cas9-Hifi and/or having a higher catalytic efficacy such as K918N SpyCECas9. Further, the term "Cas enzyme" as used herein includes a catalytically fully active Cas nuclease capable of creating a double-strand break in a DNA template, a catalytically partially active Cas nickase capable of creating a single-strand break in a double stranded DNA template, e.g. Cas9 D10A or Cas9 H840A, and a catalytically inactive Cas enzyme, e.g. dCas9. Further, the term "Cas enzyme" includes split-fusion versions of the above enzymes, e.g. split-fusion versions of Cas9 or Cas9 D10A.

In certain embodiments, the oligonucleotide targeting complex comprises a CRISPR base editor, preferably a cytosine deaminase base editor, such as APOBEC, PmCDA1 or AID, an adenine deaminase base editor, such as TadA, or a combination thereof.

In certain embodiments, the oligonucleotide targeting complex comprises a CRISPR activator, such as VP64, p65, HSF1, RTA, Tet1, p300, PRDM9, or a combination thereof.

In certain embodiments, the oligonucleotide targeting complex comprises a CRISPR repressor, preferably a KRAB domain, SALL1, SDS3, ZIM3, MeCP2, DNMT3A, LSD1, or a combination thereof.

In certain embodiments, the oligonucleotide targeting complex comprises a CRISPR prime editor, preferably PE5max, M-MLV reverse transcriptase, or a combination thereof. PE5max may be used in combination with dominant negative MLH1 to enhance prime genome editing (Chen et al., Cell 2021, 184, 5635-5652, which is incorporated herein by reference).

The oligonucleotide targeting complex of the invention further comprises and a NTA, IDA or APA-modified nucleic acid. In one embodiment, the nucleic acid is a DNA molecule, preferably a single-stranded DNA (ssDNA), a double-stranded DNA (dsDNA) or a partially double-stranded DNA. In another embodiment, the nucleic acid is a RNA molecule, preferably a single-stranded RNA, a double-stranded RNA or a partially double-stranded RNA. The nucleic acid may be any DNA or RNA molecule including modified DNA/RNA, such as chemically modified DNA/RNA including phosphorothioate bonds, for example, or base modified DNA/RNA including locked nucleic acids, 2'-O-methoxy-ethly bases, 2'-O-methyl RNA bases, and/or fluoro bases, for example, or any other modification.

In certain embodiments, the nucleic acid comprises a DNA repair template, preferably a CRISPR/Cas DNA repair template. A DNA repair template particularly serves to support HDR repair. DNA repair templates are well known in the art and particularly comprise a linear or circular DNA template sequence, which is optionally modified. Suitable modifications comprise chemical modification such as phosphorothioate bonds for improved stability of the DNA repair template, or terminal protection such as by PEG.

In a preferred embodiment, the oligonucleotide targeting complex comprises Cas9 or Cas9D10A protein and an ssDNA donor. An ssDNA donor serves to insert or change short sequences (single nucleotide polymorphisms (SNPs), substitutions, epitope tags, etc.) of DNA in an endogenous genomic target region. A typical insert length is 50 nucleotides or less. Since the ssDNA donor is arranged in spatial proximity to the Cas enzyme, HDR efficiency is improved.

In some embodiments, the oligonucleotide targeting complex may be immobilized on a substrate. The term "substrate" refers to a structure to which the complex can be coupled reversibly or irreversibly and includes, for example, planar matrices or beads. Suitable substrates are made of glass, silica, or polymeric resins. In a preferred embodiment, the nucleic acid-peptide-complex is not immobilized on a substrate.

The modified nucleic acid is a NTA, IDA or APA-modified nucleic acid as described herein. Preferably, the modified nucleic acid is a tris-NTA-modified nucleic acid, a tris-IDA-modified nucleic acid or a tris-APA-modified nucleic acid, more preferably a tris-NTA-modified nucleic acid.

The oligonucleotide targeting complex may be formed between the His-tag carried by the protein or peptide and at least one carboxylic acid (such as acetic acid) carried by the nucleic acid. A complex may be formed by interaction with a polyvalent metal ion, preferably a divalent metal ion. A suitable metal ion is a Ni(II)-ion, a Co(II)-ion, a Zn(II)-ion, a Cu(II)-ion, or a Fe(II)-ion, preferably Ni²⁺. For example, the oligonucleotide targeting complex may be a His-tag/Ni²⁺/NTA complex, a His-tag/Ni²⁺/IDA complex or a His-tag/Ni²⁺/APA complex.

Synthesis of an oligonucleotide targeting complex according to the invention may include modifying a nucleic acid molecule with NTA, IDA or APA, mixing the modified nucleic acid with a protein or peptide and polyvalent metal ions, such as Ni²⁺, to obtain the oligonucleotide targeting complex.

The oligonucleotide targeting complex may be a gene editing complex. Gene editing may comprises introducing a staggered cut into the doubled-stranded genome of a target cell or target organism, introducing a blunt-ended cut into the double-stranded genome of a target organism, insertion of a predetermined nucleotide sequence in the genome of a target cell or target organism, deletion of a predetermined nucleotide sequence in the genome of a target cell or target organism, modification of a predetermined nucleotide sequence in the genome of a target cell or target organism and combinations thereof.

A further aspect of the invention is the use of an oligonucleotide targeting complex as described herein for manufacturing a nucleic acid-peptide-complex as described herein. A nucleic acid-peptide-complex may be formed by at least two oligonucleotide targeting complexes, the nucleic acids of which are configured to form a double-stranded oligonucleotide. A nucleic acid-peptide-complex may be formed by binding of a first protein/peptide with a His-tag to a NTA, IDA or APA-modified first nucleic acid, preferably a tris-NTA-modified nucleic acid, in the presence of a polyvalent metal ion, such as Ni²⁺, to obtain a first oligonucleotide targeting complex. A second oligonucleotide targeting complex can be obtain accordingly from a second protein/peptide with a His-tag and a second modified nucleic acid, such as a tris-NTA-modified nucleic acid, that is the reverse complement to the first nucleic acid. When the oligonucleotide targeting complexes are mixed, the nucleic acid peptide-protein-complex is formed by Watson-crick base pairing of dsDNA.

A further aspect of the present invention is a library comprising a plurality of oligonucleotide targeting complexes as described herein, comprising different protein or peptide sequences and a NTA, IDA or APA-modified nucleic acid. "Comprising different protein or peptide sequences" means that each member of the library comprises a different protein or peptide sequences in comparison to the remaining members of the library. The protein or peptide sequence may be selected from the proteins and peptides disclosed herein.

Preferably, the library comprises oligonucleotide targeting complexes with only one type of nucleic acid modification, i.e. oligonucleotide targeting complexes with NTA nucleic acid modification, oligonucleotide targeting complexes with IDA nucleic acid modification, or oligonucleotide targeting complexes with APA nucleic acid modification. In certain preferred embodiments, the oligonucleotide targeting complexes of the library comprise a common DNA nucleic acid or complementary DNA nucleic acids. The library comprises at least two oligonucleotide targeting complexes, e.g. about 10 to about 10⁶ or even more.

A further aspect of the present invention is an in *vitro* or *in vivo* use of a nucleic acid-peptide-complex as described herein, an oligonucleotide targeting as described herein or a library as described herein for CRISPR mediated oligonucleotide binding and/or editing, particularly genome editing.

The term "CRISPR-mediated oligonucleotide binding and/or editing" includes binding and optionally cleaving of any DNA or RNA template, particularly a double-stranded DNA template or a single-stranded DNA template or a single-stranded RNA template, e.g. an oligonucleotide, a plasmid, a chromosome in vitro and in vivo. For example, the term includes activation and/or repression, e.g. dCas9 gene activation and/or repression, base editing, genome editing, epigenome editing and/or prime editing.

The nucleic acid-peptide-complex, oligonucleotide targeting complex and library as described herein are suitable for use in genome editing in a eukaryotic target cell, particularly in a eukaryotic target cell as described in the following, including a vertebrate target cell, e.g. an animal target cell such as a mammalian target cell, e.g. a human target cell, but also a target cell from non-human animals such as rodents, e.g. mice or zebrafish including a stem cell, e.g. human stem cell, for example an embryonic stem cell or a pluripotent stem cell. In some embodiments, the target cell is a stem cell of a eukaryotic target organism, including an induced or embryonic pluripotent stem cell such as a human induced or embryonic pluripotent stem cell but also an induced or embryonic pluripotent stem cell from non-human animals. In other embodiments, the target cell is a hematopoietic cell or a hematopoietic progenitor cell. In still other embodiments, the target cell is an immortalized cell such as a cancer cell. In still other embodiments, the target cell is a plant cell.

The nucleic acid-peptide-complex, oligonucleotide targeting complex and library as described herein are further suitable for use in genome editing in a prokaryotic target cell, such as bacterial cells, or *in vitro* in the presence of target DNA and/or RNA.

A further aspect of the present invention is a preparation comprising a nucleic acid-peptide-complex as described herein, an oligonucleotide targeting complex as described herein or a library as described herein for use in therapy including human medicine and veterinary medicine.

The use in therapy comprise a use *in vitro,* e.g. in isolated cells or cell clusters with optional implantation of the treated cell in a target organism, but also *in vivo,* i.e. in a target organism. The preparation can be applied in cell types and with genome editing procedures as indicated above, for example.

An effective dose of the compounds according to the invention, or their salts, solvates or prodrugs thereof may be used, in addition to physiologically acceptable carriers, diluents and/or adjuvants for producing a pharmaceutical preparation. The dose of the active compounds (i.e. nucleic acid-peptide-complex and/or oligonucleotide targeting complex) can vary depending on the route of administration, the age and weight of the patient, the nature and severity of the diseases to be treated, and similar factors. The daily dose can be given as a single dose, which is to be administered once, or be subdivided into two or more daily doses.

Suitable administration forms are oral, parenteral, intravenous, transdermal, topical, inhalative, intranasal and sublingual preparations. Particular preference is given to using oral, parenteral, e.g. intravenous or intramuscular, intranasal preparations, e.g. dry powder or sublingual, of the compounds according to the invention. The customary galenic preparation forms, such as tablets, sugar-coated tablets, capsules, dispersible powders, granulates, aqueous solutions, alcohol-containing aqueous solutions, aqueous or oily suspensions, syrups, juices or drops, can be used.

Solid medicinal forms can comprise inert components and carrier substances, such as calcium carbonate, calcium phosphate, sodium phosphate, lactose, starch, mannitol, alginates, gelatine, guar gum, magnesium stearate, aluminium stearate, methyl cellulose, talc, highly dispersed silicic acids, silicone oil, higher molecular weight fatty acids, (such as stearic acid), gelatine, agar agar or vegetable or animal fats and oils, or solid high molecular weight polymers (such as polyethylene glycol); preparations which are suitable for oral administration can comprise additional flavourings and/or sweetening agents, if desired.

Liquid medicinal preparations can be sterilized and/or, where appropriate, comprise auxiliary substances, such as preservatives, stabilizers, wetting agents, penetrating agents, emulsifiers, spreading agents, solubilizers, salts, sugars or sugar alcohols for regulating the osmotic pressure or for buffering, and/or viscosity regulators.

Preparations for parenteral administration can be present in separate dose unit forms, such as ampoules or vials. Use is preferably made of solutions of the active compound, preferably aqueous solution and, in particular, isotonic solutions and also suspensions. These injection forms can be made available as ready-to-use preparations or only be prepared directly before use, by mixing the active compound, for example the lyophilisate, where appropriate containing other solid carrier substances, with the desired solvent or suspending agent.

Intranasal preparations can be present as aqueous or oily solutions or as aqueous or oily suspensions. They can also be present as lyophilisates, which are prepared before use using the suitable solvent or suspending agent.

Inhalable preparations can present as powders, solutions or suspensions. Preferably, inhalable preparations are in the form of powders, e.g. as a mixture of the active ingredient with a suitable formulation aid such as lactose.

The preparations are produced, aliquoted and sealed under the customary antimicrobial and aseptic conditions.

The nucleic acid-peptide-complexes and oligonucleotide targeting complexes of the invention may be administered alone or as a combination therapy with further active agents.

The medical use of the nucleic acid-peptide-complexes and oligonucleotide targeting complexes of the invention particularly encompasses target gene therapy, e.g. the treatment of disorders associated with an undesired genotype of a patient in need of the treatment. For example, the disorder is a metabolic dysfunction or cancer. By means of the invention, cells from the patient may be subjected to a genome editing procedure in the presence of a combination as described above, thereby increasing the precise genome editing efficiency. This procedure may be carried out *in vivo,* i.e. by administering the combination to the patient or *ex vivo* with cells isolated from the patients, which are - after successful genome editing - reimplanted into the patient. The patient may be a vertebrate animal such as a mammal, preferably a human patient. Finally, the nucleic acid-peptide-complexes and oligonucleotide targeting complexes of the invention are also suitable for genome editing in plant cells or plants.

The present invention is further defined by, but not limited to, the subject matter of the following items.
1. A nucleic acid-peptide-complex comprising at least two subunits, wherein each subunit comprises
   - a protein or peptide including a His-tag, and
   - a nitrilotriacetic acid (NTA), iminodiacetic acid (IDA) or
   aminopolycarboxylic acid (APA)-modified nucleic acid,
   wherein the nucleic acids of the at least two subunits are configured to form a double-stranded oligonucleotide.
2. The nucleic acid-peptide-complex according to item 1, wherein the modified nucleic acid is a tris-NTA-modified nucleic acid, a tris-IDA-modified nucleic acid or a tris-APA-modified nucleic acid, preferably a tris-NTA-modified nucleic acid.
3. The nucleic acid-peptide-complex according to any of items 1-2, wherein the complex comprises at least one recombinant protein or peptide and preferably wherein each protein or peptide is recombinant.
4. The nucleic acid-peptide-complex according to any of items 1-3, wherein each protein or peptide is independently selected from an enzyme such as a CRISPR enzyme and variants thereof, a base editor such as a CRISPR base editor, a transcriptional activator such as a CRISPR activator, a transcriptional repressor such as a CRISPR repressor, a reverse transcriptase such as a CRISPR prime editor, an epigenic modulator, a DNA repair modulator, a splicing modulator, a fluorescent protein or a combination thereof.
5. The nucleic acid-peptide-complex according to any of items 1-4, wherein the protein or peptide of at least one subunit comprises a targetable oligonucleotide binding protein, wherein the targetable oligonucleotide protein is preferably selected from a CRISPR enzyme and variants thereof including a CRISPR enzyme nickase, a catalytically inactive CRISPR enzyme variant and an optimized CRISPR enzyme variant.
6. The nucleic acid-peptide-complex according to any of items 4-5, wherein the CRISPR enzyme and variants thereof are selected from a Cas enzyme, a Cas nickase, a catalytically inactive Cas variant and an optimized Cas variant, preferably Cas9, dCas9, Cas9-D10A, Cas9-H84A, Cas12a, Cas12b, Cas12c, Cas12d, Cas12e, Cas12f, Cas12g, Cas12h, Cas12i, Cas12j, Cas12k, Cas13a, Cas13b, Cas13c, Cas13d, Cas14, or other oligonucleotide guided nucleases including recombinant variants thereof.
7. The nucleic acid-peptide-complex according to any of items 1-6, wherein at least one nucleic acid comprises a DNA repair template, preferably a CRISPR/Cas DNA repair template.
8. The nucleic acid-peptide-complex according to any of items 1-7, wherein at least one subunit is a nucleic acid-peptide-complex and preferably wherein the at least two subunits are nucleic acid-peptide-complexes.
9. The nucleic acid-peptide-complex according to any of items 1-8, wherein each subunit further comprises a polyvalent metal ion, preferably a divalent metal ion, such as a Ni(II)-ion, a Co(II)-ion, a Zn(II)-ion, a Cu(II)-ion, or a Fe(II)-ion, preferably Ni²⁺.
10. The nucleic acid-peptide-complex according to any of items 1-9, wherein the complex comprises at least one protein including a His-tag, the protein comprising a CRISPR base editor, preferably a cytosine deaminase base editor, such as APOBEC, PmCDA1 or AID, an adenine deaminase base editor, such as TadA, or a combination thereof.
11. The nucleic acid-peptide-complex according to any of items 1-9, wherein the complex comprises at least one protein including a His-tag, the protein comprising a CRISPR activator, such as VP64, p65, HSF1, RTA, Tet1, p300, PRDM9, or a combination thereof.
12. The nucleic acid-peptide-complex according to any of items 1-9, wherein the complex comprises at least one protein including a His-tag, the protein comprising a CRISPR repressor, preferably a KRAB domain, SALL1, SDS3, ZIM3, MeCP2, DNMT3A, LSD1, or a combination thereof.
13. The nucleic acid-peptide-complex according to any of items 1-9, wherein the complex comprises at least one protein including a His-tag, the protein comprising a CRISPR prime editor, preferably PE5max, M-MLV reverse transcriptase, or a combination thereof.
14. The nucleic acid-peptide-complex according to any of items 1-13, wherein the His-tag is attached to the N-terminal of the protein or peptide.
15. The nucleic acid-peptide-complex according to any of items 1-13, wherein the His-tag is attached to the C-terminal of the protein or peptide.
16. The nucleic acid-peptide-complex according to any of items 1-13, wherein the His-tag is an internal His-tag.
17. The nucleic acid-peptide-complex according to any of items 1-6, wherein the His-tag comprises 2-14 His residues, preferably 4-10 His residues.
18. The nucleic acid-peptide-complex according to any of items 1-17, wherein the His-tag comprises a spacer positioned between the protein or peptide and the His residues.
19. The nucleic acid-peptide-complex according to any of items 1-18, wherein the His residues are arranged in a consecutive manner.
20. The nucleic acid-peptide-complex according to any of items 1-18, wherein the His residues are arranged in an alternating manner with at least one further amino acid.
21. The nucleic acid-peptide-complex according to any of items 1-20, wherein the His-tag is selected from Hiss, His₈ and His₁₀.
22. The nucleic acid-peptide-complex according to any of items 1-21, wherein the nucleic acids are DNA molecules, preferably ssDNA molecules, and particularly complementary ssDNA.
23. The nucleic acid-peptide-complex according to any of items 1-21, wherein the nucleic acids are RNA molecules, preferably single stranded RNA molecules, and particularly complementary single stranded RNA molecules.
24. The nucleic acid-peptide-complex according to any of items 1-23, wherein the nucleic acid-peptide-complex comprises 2, 3, 4 or more subunits.
25. The nucleic acid-peptide-complex according to any of items 1-24, wherein the nucleic acids of the at least two subunits form a double-stranded oligonucleotide.
26. The nucleic acid-peptide-complex according to any of items 1-25, wherein the double-stranded oligonucleotide is formed by a first nucleic acid and a second nucleic acid.
27. The nucleic acid-peptide-complex according to any of items 1-26, wherein a first subunit comprises a CRISPR enzyme protein and a second subunit comprises a protein or peptide selected from a CRISPR base editor, a CRISPR activator, a CRISPR repressor, a CRISPR prime editor, an epigenic modulator, a DNA repair modulator, a splicing modulator, or a fluorescent protein.
28. The nucleic acid-peptide-complex according to any of items 1-27, wherein the nucleic acids of the at least two subunits are configured to form a partially double-stranded oligonucleotide.
29. The nucleic acid-peptide-complex according to any of items 1-28, which is a gene editing complex.
30. The nucleic acid-peptide-complex according to any of items 1-29, wherein the nucleic acid-peptide-complex is not immobilized.
31. The nucleic acid-peptide-complex according to any of items 1-30, comprising an oligonucleotide targeting complex according to items 34-55 as a subunit.
32. The nucleic acid-peptide-complex according to any of items 1-31, wherein the APA comprises at least one tetradentate aminopolycarboxylic acid and/or at least one pentadentate aminopolycarboxylic acid.
33. The nucleic acid-peptide-complex according to any of items 1-32, wherein the APA comprises at least one EDTA, DTPA, TTHA, EDDS, EGTA, DOTA, NOTA or a combination thereof.
34. An oligonucleotide targeting complex comprising:
   - a protein or peptide including a His-tag and
   - a nitrilotriacetic acid (NTA), iminodiacetic acid (IDA) or aminopolycarboxylic acid (APA)-modified nucleic acid,
   wherein the protein or peptide is selected from a CRISPR enzyme and variants thereof, a CRISPR base editor, a CRISPR activator, a CRISPR repressor, a CRISPR prime editor, or a combination thereof.
35. The oligonucleotide targeting complex according to item 34, wherein the modified nucleic acid is a tris-NTA-modified nucleic acid, a tris-IDA-modified nucleic acid or a tris-APA-modified nucleic acid, preferably a tris-NTA-modified nucleic acid.
36. The oligonucleotide targeting complex according to any of items 34-35, wherein the protein or peptide is recombinant.
37. The oligonucleotide targeting complex according to any of items 34-36, wherein the complex comprises a protein selected from a CRISPR enzyme and variants thereof including a CRISPR enzyme nickase, a catalytically inactive CRISPR enzyme variant and an optimized CRISPR enzyme variant.
38. The oligonucleotide targeting complex according to any of items 34-37, wherein the CRISPR enzyme and variants thereof are selected from a Cas enzyme, a Cas nickase, a catalytically inactive Cas and an optimized Cas, preferably Cas9, dCas9, Cas9-D10A, Cas9-H840A, Cas12a, Cas12b, Cas12c, Cas12d, Cas12e, Cas12f, Cas12g, Cas12h, Cas12i, Cas12j, Cas12k, Cas13a, Cas13b, Cas13c, Cas13d, Cas14, or other oligonucleotide guided nucleases including recombinant variants thereof.
39. The oligonucleotide targeting complex according to any of items 34-38, wherein the nucleic acid is DNA and preferably a single-stranded DNA (ssDNA), a double-stranded DNA (dsDNA) or a partially double-stranded DNA.
40. The oligonucleotide targeting complex according to any of items 34-38, wherein the nucleic acid is RNA and preferably a single-stranded RNA, a double-stranded RNA or a partially double-stranded RNA.
41. The oligonucleotide targeting complex according to any of items 34-40, wherein the nucleic acid comprises a DNA repair template, preferably a CRISPR/Cas DNA repair template.
42. The oligonucleotide targeting complex according to any of items 34-41, wherein the complex further comprises a polyvalent metal ion, preferably a divalent metal ion, such as a Ni(II)-ion, a Co(II)-ion, a Zn(II)-ion, a Cu(II)-ion, or a Fe(II)-ion and preferably Ni²⁺
43. The oligonucleotide targeting complex according to any of items 34-42, wherein the protein comprises a CRISPR base editor, preferably a cytosine deaminase base editor, such as APOBEC, PmCDA1 or AID, an adenine deaminase base editor, such as TadA, or a combination thereof.
44. The oligonucleotide targeting complex according to any of items 34-42, wherein the protein comprises a CRISPR activator, such as VP64, p65, HSF1, RTA, Tet1, p300, PRDM9, or a combination thereof.
45. The oligonucleotide targeting complex according to any of items 34-42, wherein the protein comprises a CRISPR repressor, p preferably a KRAB domain, SALL1, SDS3, ZIM3, MeCP2, DNMT3A, LSD1, or a combination thereof.
46. The oligonucleotide targeting complex according to any of items 34-42, wherein the protein comprises a CRISPR prime editor, preferably PE5max, M-MLV reverse transcriptase, or a combination thereof.
47. The oligonucleotide targeting complex according to any of items 34-46, wherein the His-tag is attached to the N-terminal or the C-terminal of the protein or is an internal His-tag.
48. The oligonucleotide targeting complex according to any of items 34-47, wherein the His-tag comprises 2-14 His residues, preferably 4-10 His residues.
49. The oligonucleotide targeting complex according to any of items 34-48, wherein the His-tag comprises a spacer positioned between the protein or peptide and the His residues.
50. The oligonucleotide targeting complex according to any of items 34-49, wherein the His residues are arranged in a consecutive manner.
51. The oligonucleotide targeting complex according to any of items 34-49, wherein the His residues are arranged in an alternating manner with at least one further amino acid.
52. The oligonucleotide targeting complex according to any of items 34-51, wherein the His-tag is selected from Hiss, Hiss and His₁₀.
53. The oligonucleotide targeting complex according to any of items 34-52, which is a gene editing complex.
54. The oligonucleotide targeting complex according to any of items 34-53, wherein the APA comprises at least one tetradentate aminopolycarboxylic acid and/or at least one pentadentate aminopolycarboxylic acid.
55. The oligonucleotide targeting complex according to any of items 34-54, wherein the APA comprises at least one EDTA, DTPA, TTHA, EDDS, EGTA, DOTA, NOTA or a combination thereof.
56. Use of an oligonucleotide targeting complex according to any of items 34-55 for manufacturing a nucleic acid-peptide-complex according to any of items 1-33.
57. A library comprising a plurality of oligonucleotide targeting complexes according to any of items 34-55, comprising different protein or peptide sequences and a NTA, IDA or APA-modified nucleic acid, particularly a common DNA nucleic acid or complementary DNA nucleic acids.
58. *In vitro* or *in vivo* use of a nucleic acid-peptide-complex according to any of items 1-33, an oligonucleotide targeting complex according to any of items 34-55 or a library of item 57 for CRISPR mediated oligonucleotide binding and/or editing, particularly genome editing.
59. Use according to item 58 in an eukaryotic cell such as an animal cell, e.g. a mammalian cell including a human cell, or a plant cell, or in a prokaryotic cell, e.g. a bacterial cell.
60. A preparation comprising a nucleic acid-peptide-complex according to any of items 1-33, an oligonucleotide targeting complex according to any of items 34-55 or a library of item 57 for use in therapy including human medicine and veterinary medicine.

### Figure Legends

**Figure 1****: Schematic of the invention.** Coupling of a His-tagged protein/peptide such as Cas9 with a modified nucleic acid such as tris-NTA DNA to obtain an oligonucleotide targeting complex, combining at least two oligonucleotide targeting complexes to obtain a nucleic acid-protein/peptide-complex and combinatorial coupling of several proteins/peptides and/or several (partially) double-stranded complementary nucleic acid strands.

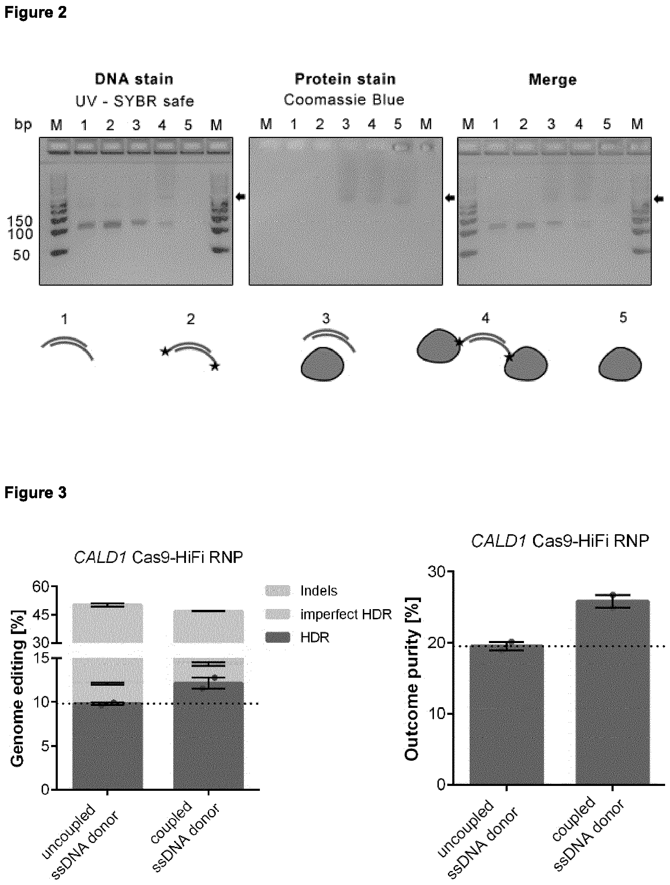

**Figure 2****: Formation of an oligonucleotide targeting complex according to the invention.** dsDNA 90bp (1), unpurified partially tris-NTA modified DNA 90bpas (2), dsDNA 90bp and Cas9-His Protein (3), unpurified partially tris-NTA modified DNA 90bp and Cas9-His protein (4), Cas9-His protein (5). A 50bp sdDNA Marker was used (M). The 4% EX-Gel SYBR Safe was imaged with UV to visualize dsDNA (left) and the same gel was afterwards stained using Coomassie Blue to visualize protein (middle). The merge of both images is shown on the right. The arrow indicates the presence of protein. Only in lane 4, where both Cas9-His and partially tris-NTA modified DNA 90bp is present, an additional DNA band at the height of protein is visible - thus indicating dsDNA coupled to Cas9-His. As the tris-NTA DNA was not purified, a predominant band of non-modified DNA that is not coupled to Cas9-His is also present.
**Figure 3****: Coupling of ssDNA-donor to Cas9-His increases HDR efficiency and outcome purity.** Genome editing frequencies for the CALD1 target in human induced pluripotent stem cells (left). HDR, mix (HDR with indels), and indels are indicated in green (lower area), light green (middle area), and blue (top area), respectively. Further, the ratio of HDR-dependent intended edits to all genome editing events that differ from the wild-type sequence was quantified (cf. 'outcome purity' at the right). Error bars show the SEM.

### Examples

### Example 1: NTA-modification of DNA oligonucleotides

DNA oligonucleotides were subjected to NTA-modification in line with Goodman et al. (ChemBioChem 2009, 10, 1551-1557). DNA oligonucleotides with three C6-amine modifications (Uni-LinkTM amino modifiers, Clontech, supplied by Integrated DNA Technologies; Table 1) were dissolved in H₂O (to 1 mM) and desalted by transferring the solution into phosphate buffer (100 mM sodium phosphate, 100 mM NaCl, pH 7.3) using a size-exclusion spin column (Bio-Rad, Micro Bio-Spin 6). DNA solution (50 µL) was combined with freshly dissolved SPDP (Sigma-Aldrich; 12.5 µL, 50mM in DMSO). After the mixture had been incubated for 1 h at room temperature, excess SPDP was removed by using a size-exclusion spin column that had been washed with phosphate buffer. TCEP (Tris(2-carboxyethyl)phosphine hydrochloride; Sigma-Aldrich; 6.25 µL, ~100 mM) was added, and the mixture was incubated for 15 min at room temperature to reduce the disulfide bonds of SPDP. Maleimide-C3-NTA (Dojindo, Kumamoto, Japan) was dissolved in phosphate buffer (50 mg mL-1). The maleimide-C3-NTA solution (7 µL) was added to the DNA solution, and the mixture was incubated for 1 h at room temperature. Excess maleimide was removed by transferring the sample into PBS (GIBCO 10010-056) by using a spin column. The samples were then stored at 4 °C.

**Table 1: DNA sequences used for tris-NTA modification**

| | |
|---|---|
| CALD1_90 nt_triA (SEQ ID NO: 12) | /5AmMC6//iUniAmM//iUniAmM/GTATACTGCTCCAGTCTGCTGTCAATCT TGGAGACTACTGCTGCTTGATGGGTCGATTTGACACCACTGCTAAAAA AGTAAACACATACA |
| CALD1_90nt_ RC_triA (SEQ ID NO: 13) | /5AmMC6//iUniAmM//iUniAmM/TGTATGTGTTTACTTTTTTAGCAGTGG TGTCAAATCGACCCATCAAGCAGCAGTAGTCTCCAAGATTGACAGC AGACTGGAGCAGTATAC |

### Example 2: Formation of DNA/Cas9 oligonucleotide targeting complexes

The NTA-modified oligonucleotides obtained in Example 1 were diluted to a concentration of 2 µM in 200 µL TN buffer (10 mM Tris, 100 mM NaCl, pH 8), hybridized by heating to 95°C for 2 min and cooled to room temperature. Single-stranded and hybridized modified oligonucleotides were combined with NiSOa (2 µL, 5 mM) and incubated for 30 min at room temperature before Cas9 (IDT) was added to a final concentration of 165 nM and incubated for 5 minutes. Unmodified oligonucleotides were treated likewise.

Samples of (1) DNA, (2) unpurified partially tris-NTA modified DNA, (3) DNA and Cas9-His protein, (4) unpurified partially tris-NTA modified DNA and Cas9-His protein, and (5) Cas9-His protein were loaded on a 4% EX-gel and ran for 10min before image acquisition. The agarose gel was fixed for 10min (fixation buffer: 40% ethanol, 10% acetic acid), washed once with deionized water and subsequently stained in colloidal Coomassie Brilliant Blue solution (QC Colloidal Coomassie Stain #1610803) for 20h. Afterwards it was destained in deionized water for 3h.

Figure 2 shows that only for sample (4), where both Cas9-His and partially tris-NTA modified DNA is present, an additional DNA band at the height of protein is visible - thus indicating dsDNA coupled to Cas9-His. As the tris-NTA DNA was not purified a predominant band of non-modified DNA that is not coupled to Cas9-His is also present.

### Example 3: Assessment of HDR efficiency and outcome purity for the CALD1 target in human induced pluripotent stem cells

HDR efficiency for the CALD1 target in human induced pluripotent stem cells by using an oligonucleotide targeting complex according to the invention was assessed as follows. Sense orientation CALD1 ssDNA donors obtained in Example 2 were used.

### Cell culture

409-B2 human induced pluripotent stem cells (GMO permit AZ 54-8452/26) that were grown on Matrigel Matrix (Corning, 35248) in mTeSR1 medium (StemCell Technologies, 05851) with supplement (StemCell Technologies, 05852) and MycoZap Plus-CL (Lonza, VZA-2011) that was replaced daily were used. At ~80% confluency, adherent cells were dissociated using EDTA (VWR, 437012 C) and split 1:6 to 1:10 in medium supplemented with 10 µM Rho-associated protein kinase (ROCK) inhibitor Y-27632 (Calbiochem, 688000) for one day after replating. Cells were grown at 37 °C in a humidified incubator with 5% CO2.

### Electroporation of oligonucleotides and ribonucleoproteins

Electroporation of oligonucleotides was done using the B-16 program of the Nucleofector 2b Device (Lonza) in cuvettes for 100 µl Human Stem Cell nucleofection buffer (Lonza, VVPH-5022), containing 0.1mM NiSO₄, 1 million cells, 78 pmol electroporation enhancer, and 320 pmol of gRNA (crRNA/tracRNA duplex from IDT, CALD1_gRNA_20mer: TGGAGACTATTGCTGCTTGA), as well as 252 pmol S.p. HiFi Cas9 (IDT) and 200 pmol of single-stranded DNA donor. Prior to electroporation, 2.8 µl PBS, 3.2µl gRNA and 4µl Cas9 were incubated at room temperature for 30 min to form a ribonucleotide complex, before addition of DNA oligonucleotides and 2 µl NiSOa (5 mM) and further incubation at room temperature for 10 min.

### Illumina library preparation and sequencing

Three days after transfection cells were detached using TrypLE (ThermoFisher, 12605036), pelleted, and resuspended in 15 µl QuickExtract (Lucigen, QE0905T). Incubation at 65 °C for 10 min, 68 °C for 5 min, and 98 °C for 5 min was performed to yield single stranded DNA. Primers containing adapter overhangs for Illumina sequencing (forward ACACTCTTTCCCTACACGACGCTCTTCCGATCTGCTAATCAGCTAGCATATGTATGAG AA (SEQ ID NO: 14), reverse GTGACTGGAGTTCAGACGTGTGCTCTTCCGATCTTTGGACTTGATTATTGTCCTAAGT G (SEQ ID NO:15)) were used to amplify each locus in a T100 Thermal Cycler (Bio-Rad) using the KAPA2G Robust PCR Kit (SIGMA, KK5024) with buffer B and 3 µl of cell extract in a total volume of 25 µl. The thermal cycling profile of the PCR was: 95 °C 3 min; 34x (95 °C 15 s, 65 °C 15 s, 72 °C 15 s); 72 °C 60 s. P5 and P7 Illumina adapters with sample specific indices were added in a subsequent PCR reaction (Kircher et al. 2012, Nucleic Acids Res. 40, e3) using Phusion HF MasterMix (Thermo Scientific, F-531L) and 0.3 µl of the first PCR product. The thermal cycling profile of the second PCR was: 98 °C 30 s; 25× (98 °C 10 s, 58 °C 10 s, 72 °C 20 s); 72 °C 5 min.

Amplifications were verified by size separating agarose gel electrophoresis using 2% EX gels (Invitrogen, G4010-11). The indexed amplicons were purified using solid phase reversible immobilization (SPRI) beads (Meyer and Kircher 2010, Cold Spring Harb. Protoc. 2010, pdb prot5448). Double-indexed libraries were sequenced on a MiSeq (Illumina) yielding 2 ×150bp (+7 bp index). After base calling using Bustard (Illumina) adapters were trimmed using leeHom (Renaud et al. 2014, Nucleic Acids Res. 42, e141).

### Amplicon sequence analysis

Bam-files were demultiplexed and converted into fastq files using SAMtools (Li et al. 2009, Bioinformatics 25, 2078-2079). CRISPResso (Pinello et al. 2016, Nat. Biotechnol. 34, 695-697) was used to analyze fastq files for percentage of wild type and indel sequences. For analysis of precise insertion frequency of 'worst-case' targets in single cell-derived cellular clones, the expected amplicon was provided to CRISPResso to call the HDR event. Analysis was restricted to amplicons with a minimum of 70% similarity to the wild type sequence and to a window of 20 bp from each gRNA. Unexpected substitutions were ignored as putative sequencing errors.

### Results

By using an oligonucleotide targeting complex of an ssDNA-donor and Cas9, genome editing was performed with an around 25% increased amount of HDR repair (12.2 %) in comparison to the use of an uncoupled ssDNA donor and Cas9 (9.8 % HDR repait). Further, the ratio of HDR-dependent intended edits to all genome editing events that differ from the wild-type sequence was quantified (outcome purity in %). The oligonucleotide targeting complex of the present invention allows for a significantly increase of the outcome purity to 25.8% in comparison to an outcome purity of 19.5 % for genome editing with separate ssDNA-donor and Cas9 molecules.

Since tris-NTA modified DNA was used as synthesized and was not purified from unmodified DNA prior to oligonucleotide targeting complex formation, the HDR-increase is due to only a fraction of tris-NTA modified DNA-donors (see Fig. 2, lane 4 unmodified DNA band vs modified DNA band). Thus, HDR efficiency can be further increased by using pure tris-NTA-DNA donor.

## Claims

1. A nucleic acid-peptide-complex comprising at least two subunits, wherein each subunit comprises
- a protein or peptide including a His-tag, preferably a recombinant protein or peptide including a His-tag, and
- a nitrilotriacetic acid (NTA), iminodiacetic acid (IDA) or
aminopolycarboxylic acid (APA)-modified nucleic acid, preferably a tris-NTA-modified nucleic acid,
wherein the nucleic acids of the at least two subunits are configured to form a double-stranded oligonucleotide.

2. The complex of claim 1, wherein each protein or peptide is independently selected from an enzyme such as a CRISPR enzyme and variants thereof, a base editor such as a CRISPR base editor, a transcriptional activator such as a CRISPR activator, a transcriptional repressor such as a CRISPR repressor, a reverse transcriptase such as a CRISPR prime editor, an epigenic modulator, a DNA repair modulator, a splicing modulator, a fluorescent protein or a combination thereof.

3. The complex according to any of claims 1-2, wherein the protein or peptide of at least one subunit comprises a targetable oligonucleotide binding protein, wherein the targetable oligonucleotide protein is preferably selected from a CRISPR enzyme and variants thereof including a CRISPR enzyme nickase, a catalytically inactive CRISPR enzyme variant and an optimized CRISPR enzyme variant.

4. The complex according to any of claims 1-3, wherein each subunit further comprises a polyvalent metal ion, preferably a divalent metal ion, such as a Ni(II)-ion, a Co(II)-ion, a Zn(II)-ion, a Cu(II)-ion, or a Fe(II)-ion, preferably Ni²⁺.

5. The complex according to any of claims 1-4, wherein the His-tag is a N-terminally attached His-tag, a C-terminally attached His-tag or an internal His-tag, and/or is preferably selected from Hiss, His₈ and His₁₀.

6. The complex according to any of claims 1-5, wherein the nucleic acids are DNA molecules, preferably ssDNA molecules, and particularly complementary ssDNA, or wherein the nucleic acids are RNA molecules, preferably ssRNA molecules, and particularly complementary ssRNA molecules.

7. The nucleic acid-peptide-complex according to any of claims 1-6, wherein at least one nucleic acid comprises a DNA repair template, preferably a CRISPR/Cas DNA repair template.

8. The nucleic acid-peptide-complex according to any of claims 1-7, wherein at least one subunit is a nucleic acid-peptide-complex and preferably wherein the at least two subunits are nucleic acid-peptide-complexes.

9. An oligonucleotide targeting complex comprising:
- a protein or peptide including a His-tag, preferably a recombinant protein or peptide, and
- a nitrilotriacetic acid (NTA), iminodiacetic acid (IDA) or aminopolycarboxylic acid (APA)-modified nucleic acid, preferably a tris-NTA-modified nucleic acid,
wherein the protein or peptide is selected from a CRISPR enzyme and variants thereof, a CRISPR base editor, a CRISPR activator, a CRISPR repressor, a CRISPR prime editor, or a combination thereof.

10. An oligonucleotide targeting complex according to claim 9, wherein the nucleic acid is DNA and preferably a ssDNA, a dsDNA or a partially double-stranded DNA, or wherein the nucleic acid is RNA and preferably a ssRNA, a dsRNA or a partially double-stranded RNA.

11. An oligonucleotide targeting complex according to any of claims 9-10, wherein the complex further comprises a polyvalent metal ion, preferably a divalent metal ion, such as a Ni(II)-ion, a Co(II)-ion, a Zn(II)-ion, a Cu(II)-ion, or a Fe(II)-ion and preferably Ni²⁺.

12. Use of an oligonucleotide targeting complex according to any of claims 9-11 for manufacturing a nucleic acid-peptide-complex according to any of claims 1-8.

13. A library comprising a plurality of oligonucleotide targeting complexes according to any of claims 9-11, comprising different protein or peptide sequences and a NTA, IDA or APA-modified nucleic acid, particularly a common DNA nucleic acid or complementary DNA nucleic acids.

14. *In vitro* or *in vivo* use of a nucleic acid-peptide-complex according to any of claims 1-8, an oligonucleotide targeting complex according to any of claims 9-11 or a library of claim 13 for CRISPR mediated oligonucleotide binding and/or editing, particularly genome editing.

15. A preparation comprising a nucleic acid-peptide-complex according to any of claims 1-8, an oligonucleotide targeting complex according to any of claims 9-11 or a library of claim 13 for use in therapy including human medicine and veterinary medicine.
